# EUROPEAN PATENT APPLICATION

(11) **EP 4 455 652 A1**
(43) Date of publication of application: **30.10.2024**
(21) Application number: 22910775.0
(22) Date of filing: 29.11.2022
(51) Int. Cl.: G01N 23/2251, G01N 21/17

(54) **PHYSICAL PROPERTY VALUE PREDICTION METHOD AND PHYSICAL PROPERTY VALUE PREDICTION SYSTEM**

(30) Priority: 21.12.2021 JP 2021206969
(71) Applicant: MEC COMPANY., LTD., Amagasaki-shi, Hyogo 660-0822 (JP)
(72) Inventor: AKAGI,Masako, Amagasaki-shi, Hyogo 660-0822 (JP)
(74) Representative: TBK
(86) International application number: PCT/JP2022/043910
(87) International publication number: WO 2023/120057

(57) **Abstract**

The physical property value prediction method include: inputting a plurality of prediction target images for each of which a measured value of the physical property value is known into a machine-learned prediction model 21, and outputting a predicted value and a feature map of each of the plurality of prediction target images; identifying, on a basis of prediction results from images almost identical in measured physical property value among the plurality of prediction target images, a poor prediction image G3 with an error between the measured value and the predicted value greater than or equal to a first threshold and a good prediction image G4 with an error between the measured value and the predicted value less than or equal to a second threshold, the second threshold being smaller than the first threshold; and extracting a feature group representing a factor in poor prediction on a basis of a difference between a frequency distribution of a plurality of features constituting the feature map of the poor prediction image G3 and a frequency distribution of a plurality of features constituting the feature map of the good prediction image G4.

## Description

### TECHNICAL FIELD

The present disclosure relates to a physical property value prediction method and a physical property value prediction system using machine learning.

### BACKGROUND ART

An electronic substrate has a laminated structure of a metal such as copper and a resin, and the quality of the electronic substrate corresponds to the adhesion between the metal and the resin. It is possible to evaluate a shape of a roughened metal surface subjected to surface treatment with a chemical to evaluate the adhesion of the metal of the electronic substrate. One of the evaluation items is, for example, measuring peel strength, but involving significant effort.

For example, Patent Document 1 discloses estimating a physical property value of a rubber material using machine learning.

### PRIOR ART DOCUMENT

### PATENT DOCUMENT

Patent Document 1: JP-A-2021-60457

### SUMMARY OF THE INVENTION

### PROBLEMS TO BE SOLVED BY THE INVENTION

Physical property value prediction based on machine learning has a problem that in a case where a black-box prediction model such as a neural network is used, on what basis and how the prediction is made and a cause of good or poor prediction accuracy are unclear.

The present disclosure provides a physical property value prediction method and a physical property value prediction system that provide a possibility of giving an explanation of prediction accuracy of a prediction model that predicts a physical property value of a material using machine learning.

### MEANS FOR SOLVING THE PROBLEMS

According of the present disclosure, there is provided a physical property value prediction method comprising: inputting, into a prediction model including a feature map output unit configured to output a feature map on a basis of an image obtained by imaging a material and a conversion unit configured to convert the feature map into a physical property value of the material, and machine-learned to receive input of the image obtained by imaging the material as an explanatory variable and output the physical property value, a plurality of prediction target images for each of which a measured value of the physical property value is known and outputting a predicted value and a feature map of each of the plurality of prediction target images; identifying, on a basis of prediction results from images almost identical in measured physical property value among the plurality of prediction target images, a poor prediction image with an error between the measured value and the predicted value greater than or equal to a first threshold and a good prediction image with an error between the measured value and the predicted value less than or equal to a second threshold, the second threshold being smaller than the first threshold; and extracting a feature group representing a factor in poor prediction on a basis of a difference between a frequency distribution of a plurality of features constituting the feature map of the poor prediction image and a frequency distribution of a plurality of features constituting the feature map of the good prediction image.

### BRIEF DESCRIPTION OF THE DRAWINGS

Fig. 1 is a block diagram illustrating a prediction system and a learning system of a first embodiment.
Fig. 2 is a diagram illustrating an example of an SEM image obtained by imaging a copper foil subjected to chemical treatment with an electron microscope.
Fig. 3 is a diagram obtained by plotting data of combinations of predicted values and measured values of Example 1.
Fig. 4 is a flowchart illustrating processing to be executed by the prediction system of the first embodiment.
Fig. 5 is a block diagram illustrating a learning system 1 and a prediction system 2 of a second embodiment.
Fig. 6 is a diagram obtained by plotting data of combinations of predicted values and measured values, and is a diagram illustrating an example where a poor prediction image and a good prediction image are identified.
Fig. 7 is a diagram illustrating the poor prediction image (SEM image).
Fig. 8 is a diagram illustrating the good prediction image (SEM image).
Fig. 9 is a diagram illustrating, as an image, a feature map obtained from the poor prediction image (SEM image).
Fig. 10 is a diagram illustrating, as an image, a feature map obtained from the good prediction image (SEM image).
Fig. 11 is a diagram illustrating a frequency distribution (histogram) of a plurality of features constituting the feature map of the poor prediction image and a frequency distribution (histogram) of a plurality of features constituting the feature map of the good prediction image.
Fig. 12 is a diagram illustrating only a feature group extracted from the feature map of the poor prediction image.
Fig. 13 is a diagram illustrating only a feature group extracted from the feature map of the good prediction image.
Fig 14 is a diagram illustrating an example of a superimposed image showing the position of the extracted feature group overlaid on the poor prediction image.
Fig 15 is a diagram illustrating an example of a superimposed image showing the position of the extracted feature group overlaid on the good prediction image.
Fig. 16 is a flowchart illustrating processing to be executed by the prediction system of the second embodiment.

### MODE FOR CARRYING OUT THE INVENTION

### <First embodiment>

Hereinafter, a first embodiment of the present disclosure will be described with reference to the drawings.

### [Learning system, prediction system]

A prediction system 2 (device) of the first embodiment predicts, using a prediction model 21, a physical property value (for example, peel strength) of a metal (copper) subjected to surface treatment with a chemical on the basis of an image obtained by imaging a surface of the metal. A learning system 1 (device) builds, using training data, the prediction model 21 on the basis of machine learning.

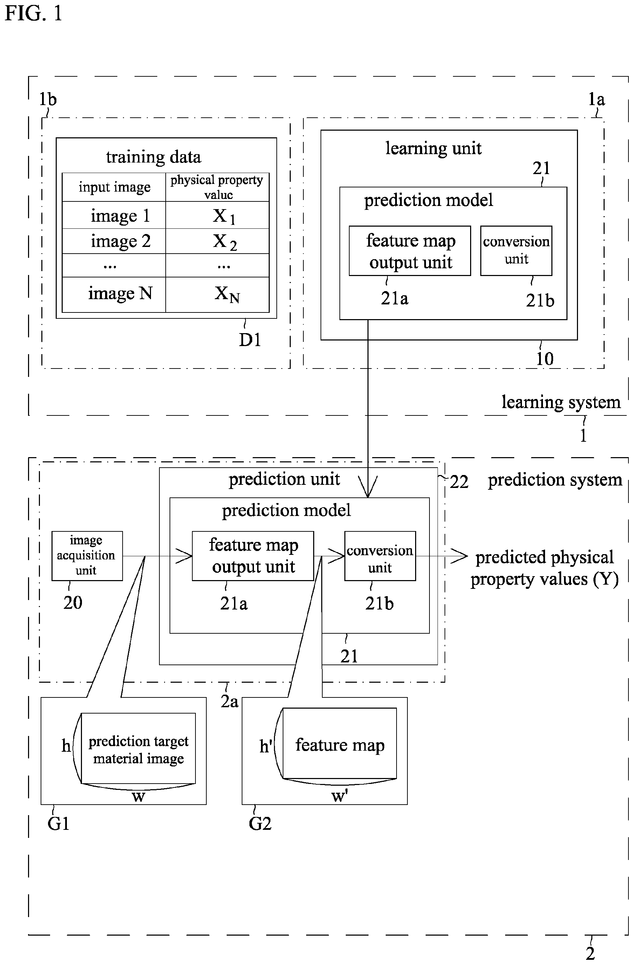

As illustrated in Fig. 1, the prediction system 2 includes an image acquisition unit 20 and a prediction unit 22. The learning system 1 includes training data D1 and a learning unit 10 that trains the prediction model 21. The training data D1 is stored in a memory 1b. The learning unit 10 is implemented by a processor 1a. The image acquisition unit 20 and the prediction unit 22 are implemented by a processor 2a. In the first embodiment, the processors 1a and 2a in one device implement each unit, but the present disclosure is not limited to such a configuration. For example, each processing may be distributed using a network, and a plurality of processors may each execute processing of the corresponding unit. That is, one or more processors execute processing.

The image acquisition unit 20 acquires an image G1 obtained by imaging a prediction target material. In the first embodiment, the image acquisition unit 20 acquires an SEM image (grayscale image) obtained by imaging a surface of a metal (copper) subjected to surface treatment with an electron microscope. The image G1 obtained by imaging the prediction target material is image data of vertical pixels h × horizontal pixels w.

The prediction model 21 is a model built, on the basis of machine learning, using the training data D 1 in which images (for example, SEM images or camera images) obtained by imaging the material and physical property values (for example, peel strength) of the material are associated with each other so as to receive the input of an image as an explanatory variable and output the physical property value of a material. The learning unit 10 of the learning system 1 updates parameters of the prediction model 21 so to make a prediction result equal to the measured value of the training data. In Fig 1, in the training data D1, input images 1 to N (N represents the number of training images) and physical quantities (X₁, X₂, ..., X_{N}) that are measured values corresponding to the input images 1 to N are associated with each other.

Although various models can be used as the prediction model 21, in the first embodiment, the prediction model 21 includes a feature map output unit 21a that outputs a feature map G2 on the basis of an input image, and a conversion unit 21b that converts the feature map G2 into a physical property value (Y). In the first embodiment, UNet is used as the feature map output unit 21a. UNet is a U-shaped convolutional neural network (CNN)-based learning model. The conversion unit 21b uses global average pooling (GAP). The feature map G2 is data containing the number of pixels obtained by multiplying the number of pixels h' corresponding to the vertical pixels h of the input image G1 by the number of pixels w' corresponding to the horizontal pixels w of the input image, and each pixel has a feature of the physical property value of the material. The conversion unit 21b converts the feature map G2 into a single physical quantity (Y). Here, "corresponding to" covers a case where the feature map is an image identical in size to the original input image and a case where the feature map is an image different in size from the original input image but identical in aspect ratio, and means that it is possible to enlarge the feature map so as to make the feature map identical in size to the original input image.

### EXAMPLES

### [Example 1]

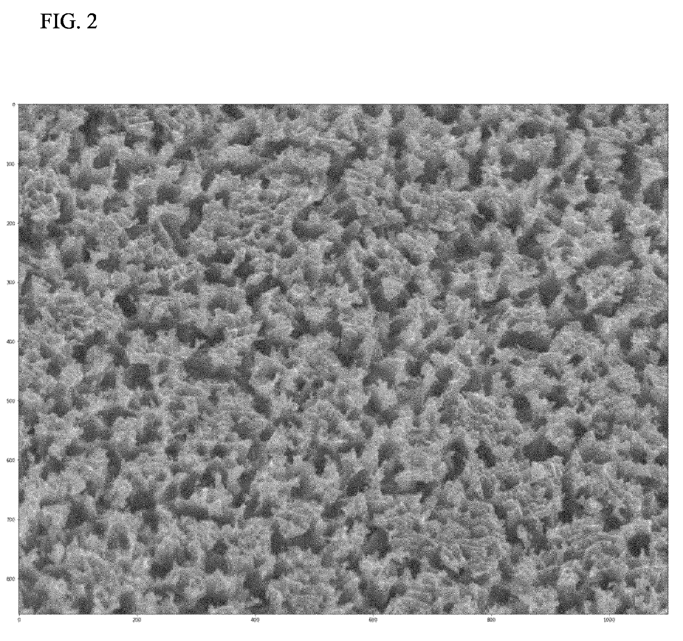

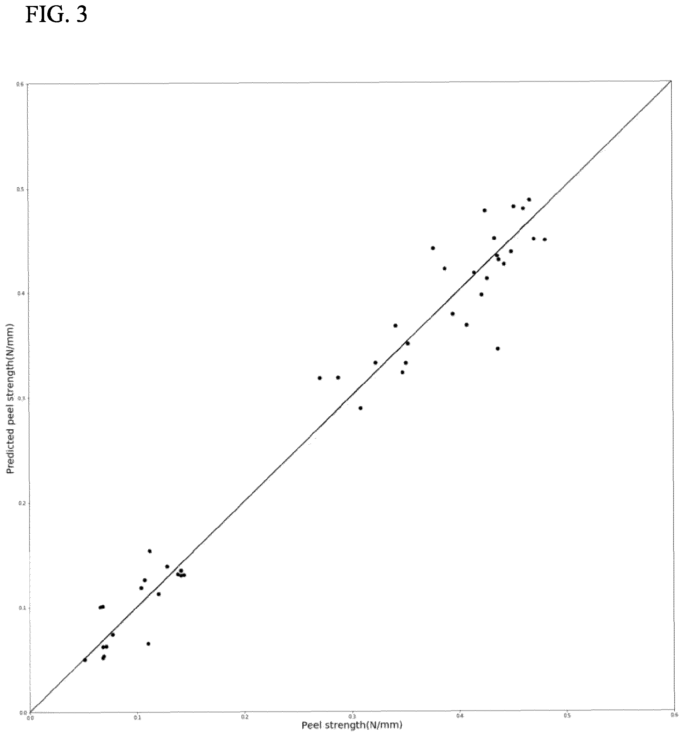

Example 1 is an example where the input image G1 is an SEM image obtained by imaging a copper foil subjected to chemical treatment with an electron microscope. Fig. 2 illustrates an example of the SEM image. The imaging conditions for the SEM image are a magnification of 3500x and a tilt angle of 45 degrees. The physical quantity was peel strength [N/mm]. This is an example where a copper surface is treated using a chemical A. 90 peel strengths were measured for 90 images. Half of the 90 pieces of data were used as data for training, and the remaining half were used as data for prediction. The mean squared error between the measured peel strengths and the predicted values was 0.0008. Fig. 3 is a diagram in which the horizontal axis represents measured peel strength and the vertical axis represents predicted peel strength, obtained by plotting data of combinations of predicted values and measured values.

### [Example 2]

In Example 2, the SEM image was used as the input image G1 as in Example 1. This is an example where a copper surface is treated using a chemical B. 72 peel strengths were measured for 72 images. Half of the 72 pieces of data were used as data for training, and the remaining half were used as data for prediction. The mean squared error between the measured peel strengths and the predicted values was 0.0012. The imaging conditions for the SEM image are the same as in Example 1.

### [Example 3]

In Example 3, the SEM image was used as the input image G1 as in Example 1. This is an example where a copper surface is treated using a chemical C. 39 peel strengths were measured for 39 images. Half of the 39 pieces of data were used as data for training, and the remaining half were used as data for prediction. The mean squared error between the measured peel strengths and the predicted values was 0.0021. The imaging conditions for the SEM image are the same as in Example 1.

### [Example 4]

Example 4 is an example where a plurality of physical property values are estimated from one SEM image that is the SEM image of Example 3. Specifically, setting the output of one UNet to a plurality of classes allows the same UNet to output (calculate) a plurality of physical property values. The plurality of physical property values each represent peel strength and roughness parameters (Sdr, Sdq). A first UNet for predicting peel strength, a second UNet for predicting Sdr, and a third UNet for predicting Sdq are parallelized. The mean squared error of peel strength was 0.001, the mean squared error of Sdr was 0.0003, and the mean squared error of Sdq was 0.0868.

### [Example 5]

Example 5 is an example where a copper surface is treated using a chemical D. The input image G1 is not the SEM image but a camera image captured by an optical camera. The camera image is subjected to only processing of separating a black background. RGB components contained in the camera image are separated into three monochrome images, and the three monochrome images are input into the prediction model 21. The physical property value was surface roughness Ra (arithmetic mean). 960 sets of data are used, half are used as training data, and the remaining half are used as prediction data. The mean squared error was 0.0153.

Note that it is also possible to convert a camera image containing RGB components into a grayscale image and input the intensity (lightness) of the grayscale image into the prediction model 21. Since there was no significant difference between the case of inputting the grayscale image and the case of inputting the three monochrome images of the RGB components, the three monochrome images of the RGB components were used in Example 5.

### [Example 6]

In Example 6, the camera image is used as the input image G1 as in Example 5. This is an example where a copper surface is treated using the chemical D. The physical property value was CIE 1976 lightness index L*. 320 sets of data are used, half are used as training data, and the remaining half are used as prediction data. The mean squared error was 11.05. L* adheres to JIS Z 8781-4.

### [Example 7]

In Example 7, the camera image is used as the input image G1 as in Example 5. This is an example where a copper surface is treated using the chemical D. The physical property value was a color coordinate a* in the CIE 1976 color space. 320 sets of data are used, half are used as training data, and the remaining half are used as prediction data. The mean squared error was 0.0062. a* adheres to JIS Z 8781-4.

### [Example 8]

In Example 8, the camera image is used as the input image G1 as in Example 5. This is an example where a copper surface is treated using the chemical D. The physical property value was a color coordinate b* in the CIE 1976 color space. 320 sets of data are used, half are used as training data, and the remaining half are used as prediction data. The mean squared error was 0.1294. b* adheres to JIS Z 8781-4.

### [Method for predicting physical property value of material]

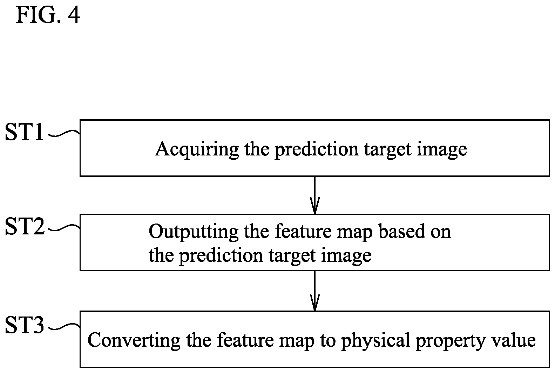

A method for predicting a physical property value of a material that is executed by the prediction system 2 will be described with reference to Fig. 4.

First, in step ST1, the image acquisition unit 20 acquires a prediction target image obtained by imaging a prediction target material. In the first embodiment, an SEM image or a camera image is acquired. In subsequent steps ST2 and ST3, the acquired prediction target image is input into the prediction model 21, and the physical property value of a material is output. Specifically, in step ST2, the feature map output unit 21a receives the input of the prediction target image and outputs the feature map G2. In step ST3, the conversion unit 21b converts the feature map G2 into the physical property value of the material.

### <Modification>

(1-1) In the embodiment illustrated in Fig. 1, the prediction model 21 includes the feature map output unit 21a that outputs the feature map G2, but may be a model that outputs a physical property value without outputting a feature map. For example, ResNet, which is a type of neural network, may be used. It goes without saying that any nonlinear model capable of processing images can be used.

(1-2) In the above-described embodiment, the prediction target material whose physical property value is to be predicted corresponds to a surface of a metal (copper) subjected to surface treatment by means of chemical reaction treatment with a chemical (etchant, polishing solution (chemical, electrolytic)) or the like, but is not limited to such a surface as long as the material has a uniform and fine surface shape. For example, the prediction target material may be a surface of a metal subjected to surface treatment by means of machining processing in which an external force such as polishing, rolling, or laser is applied. The prediction target material may be a surface coated with a coating material in which various pigments are dispersed and mixed. Further, in this case, the form (liquid, powder, etc.) of the coating material is not limited. The prediction target material may be a surface of a metal plated by electroplating, electroless plating, or the like. The prediction target material may be a surface of a film subjected to molding processing with an additive added and dispersed. The prediction target material may be a paper surface having a coated layer for receiving ink or a coated layer that provides other functionality. The material is not limited to a specific material, but may be a surface of a material subjected to calender molding, embossing molding, or the like.

(1-3) In the above-described embodiment, the peel strength has been given as the physical property value of a material, but the physical property value is not limited to the peel strength. For example, the physical property value of a material may be a physical property value related to adhesion, bonding, airtightness, water repellency, oil repellency, an antifouling property, a sliding property, a surface property (gloss, roughness), color tone, texture, a thermal property, an antibacterial property, or transmission loss.

(1-4) In the above-described embodiment, the metal surface image acquired by the SEM and the camera has been given as the material image, but the material image is not limited to such an image. Data that can be captured as an image, such as a waveform, a spectrum, a mapping image, and a numerical value, may also be provided as the material image. Examples of the data include a spectrum obtained by means of microspectroscopy (infrared, Raman, UV-Vis, etc.), energy dispersive X-ray spectroscopy (SEM-EDX), or ultrasonic testing used for non-destructive testing, and a mapping image using the spectrum.

As described above, the physical property value prediction method of the first embodiment may be executed by one or more processors, and may include: acquiring a prediction target image G1 of a prediction target material; inputting the prediction target image G1 into a prediction model machine-learned so as to receive an input of the image of the material as an explanatory variable and output the physical property value (Y) of the material, and outputting the physical property value (Y) of the material appearing in the prediction target image G1 as a predicted value.

As described above, since the physical property value of the material (metal) appearing in the prediction target image G1 can be predicted using the machine-learned prediction model 21, it is possible to reduce the monetary cost and the time cost as compared with a case where the physical property value is measured through a test or the like.

Although not particularly limited, as in the first embodiment, the prediction model 21 may include the feature map output unit 21a that outputs the feature map G2 on the basis of the input image G1, and the conversion unit 21b that converts the feature map G2 into a predicted value.

This allows the predicted value to be obtained from the feature map G2, which enables an explanation of the basis for the prediction made by the prediction model 21 to be given with the feature map G2.

Although not particularly limited, as in the first embodiment, the material to be imaged may be any one of a surface of a metal subjected to surface treatment, a surface coated with a coating material, a surface of a plated metal, a surface of a film, a paper surface, or a surface of a molded material. The above-described examples are preferred examples.

The system according to the first embodiment includes one or more processors that execute the above-described method.

The program according to the first embodiment is a program that causes one or more processors to execute the above-described method.

It is also possible to achieve the effects of the above-described method by executing such a program.

Although the embodiment of the present disclosure has been described above with reference to the drawings, it should be understood that specific configurations are not limited to the embodiment. The scope of the present disclosure is defined not only by the description of the above-described embodiment but also by the claims, and further includes equivalents of the claims and all modifications within the scope.

### <Second embodiment>

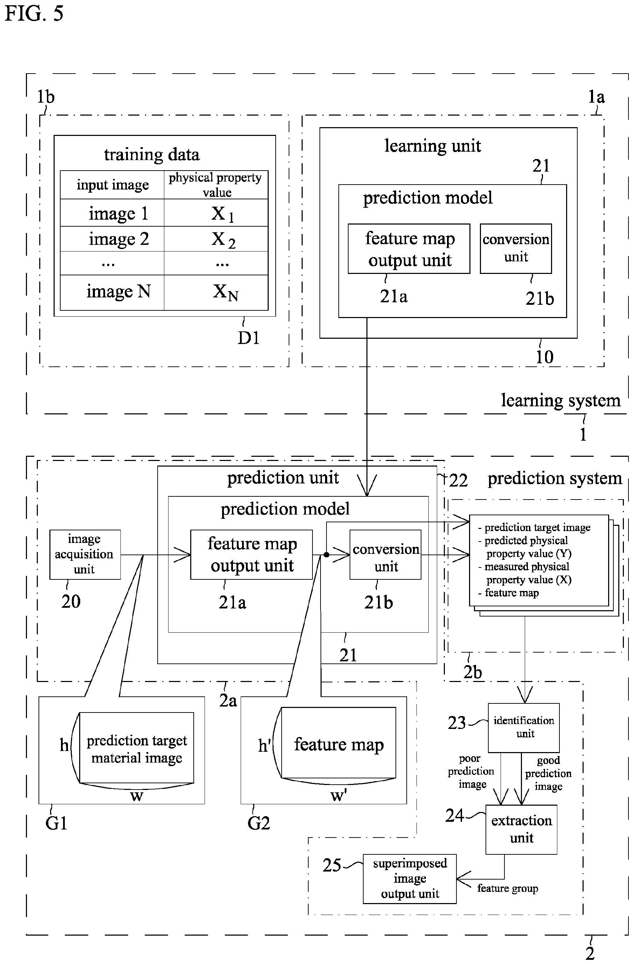

Fig. 5 is a block diagram illustrating a learning system 1 and a prediction system 2 according to a second embodiment. The second embodiment is different from the first embodiment in the configuration of the prediction system 2. Specifically, the prediction system 2 of the second embodiment is different from the prediction system 2 of the first embodiment in that an identification unit 23, an extraction unit 24, and a superimposed image output unit 25 are additionally provided. The identification unit 23, the extraction unit 24, and the superimposed image output unit 25 are implemented by the processor 2a.

As illustrated in Fig. 5, the memory 2b stores a plurality of prediction target images and physical quantities (X) that are known measured values of the material appearing in each of the prediction target images with the prediction target images and the physical quantities (X) associated with each other. The image acquisition unit 20 inputs each of the plurality of prediction target images stored in the memory 2b into the prediction model 21 (the feature map output unit 21a and the conversion unit 21b). The predicted physical property value (Y) calculated by the prediction model 21 and the feature map output by the feature map output unit 21a are stored in the memory 2b in association with the prediction target image and the measured physical property value (X).

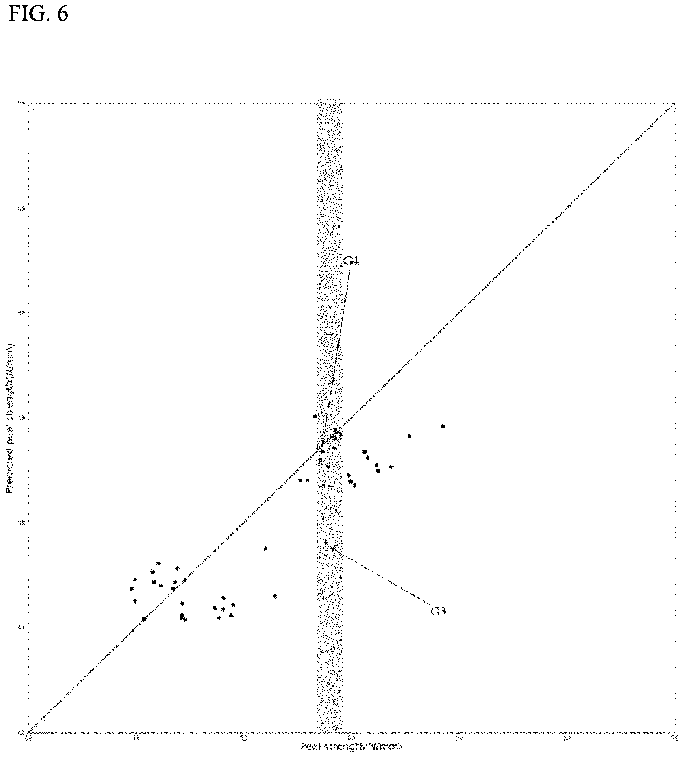

The identification unit 23 illustrated in Fig. 5 identifies a poor prediction image and a good prediction image on the basis of prediction results from images almost identical in measured physical property value among the plurality of prediction target images stored in the memory 2b. The poor prediction image is an image with an error between the measured value and the predicted value greater than or equal to a first threshold. Fig. 6 is a diagram obtained by plotting data of combinations of predicted values and measured values of a prediction target image group different from the prediction target image group illustrated in Fig. 3, where the horizontal axis represents a measured peel strength [N/mm] and the vertical axis represents a predicted peel strength [N/mm]. As illustrated in Fig. 6, points where the predicted physical property value (predicted peel strength) and the measured physical property value (measured peel strength) are equal to each other are indicated by a slanted line, data close to the slanted line (plot point) is a good prediction image, and data far from the slanted line (plot point) is a poor prediction image. The prediction accuracy for each prediction target image can be expressed by the length of a perpendicular from the corresponding plot point to the slanted line. The longer the length of the perpendicular, the larger the difference between the predicted physical property value and the measured physical property, which can be determined to be a poor prediction image, and the shorter the length of the perpendicular, the smaller the difference between the predicted physical property value and the measured physical property value, which can be determined to be a good prediction image. A good prediction image relatively close to the slanted line and a poor prediction image relatively far from the slanted line may be selected with human eyes, but automatic selection by a program is also possible. The identification unit 23 is capable of executing processing of identifying an image with an error (the length of the perpendicular) between the measured value and the predicted value is greater than or equal to the first threshold as a poor prediction image and identifying an image with an error (the length of the perpendicular) between the measured value and the predicted value is less than or equal to a second threshold as a good prediction image. The second threshold is smaller than the first threshold. The first threshold value and the second threshold value may be constants or variables. For example, the first threshold and the second threshold may be set on the basis of the largest error among the errors between the measured values and the predicted values. The first threshold may be set less than or equal to 100% and greater than or equal to 90% of the largest error, and the second threshold may be set greater than or equal to 0% and less than or equal to 10% of the largest error. Further, an image with the largest error between the measured value and the predicted value may be identified as a poor prediction image, and an image with the smallest error between the measured value and the predicted value may be identified as a good prediction image.

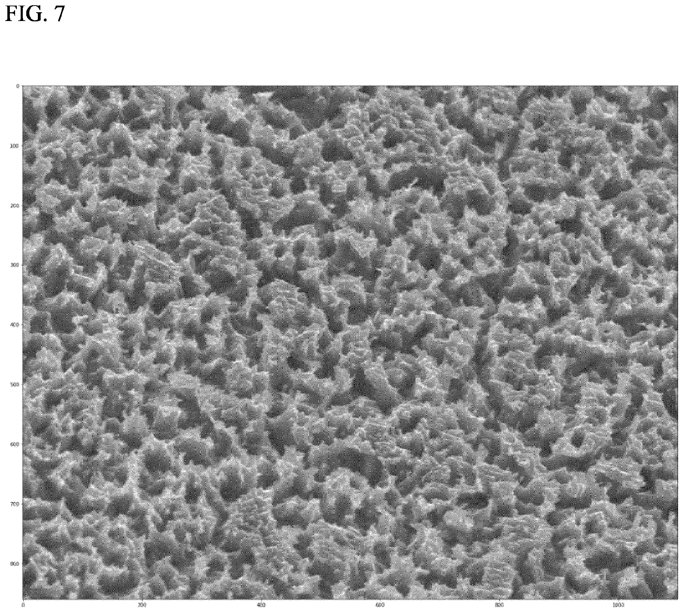

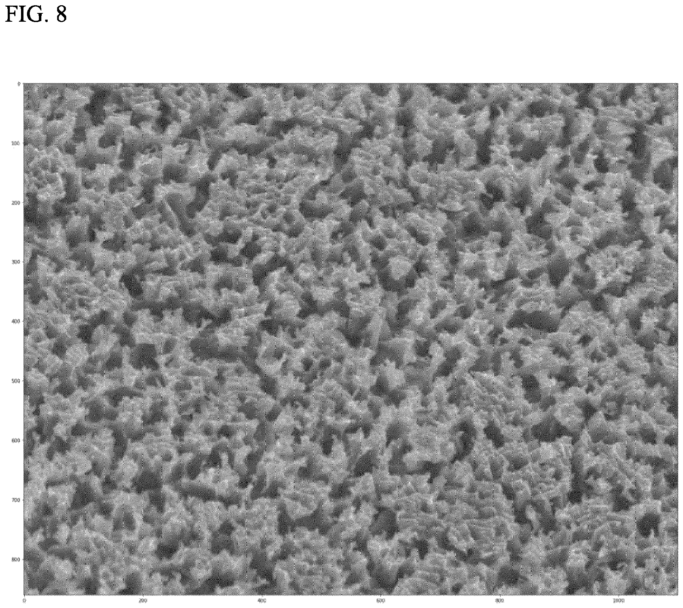

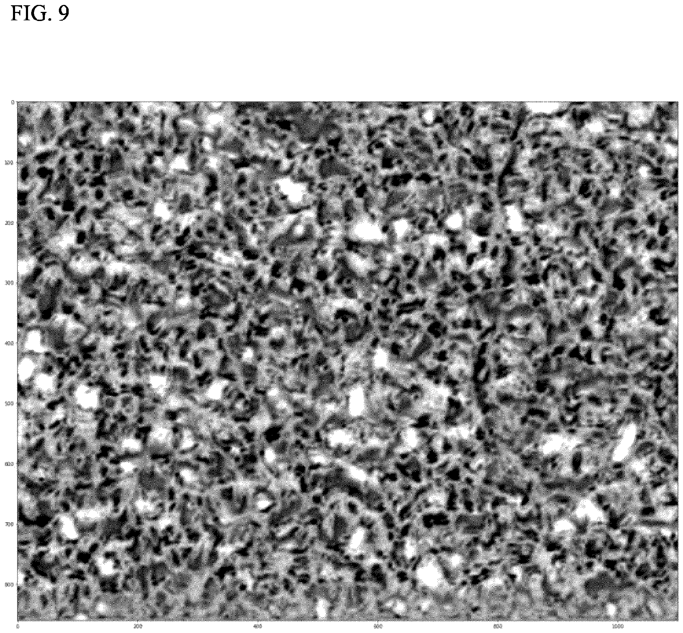

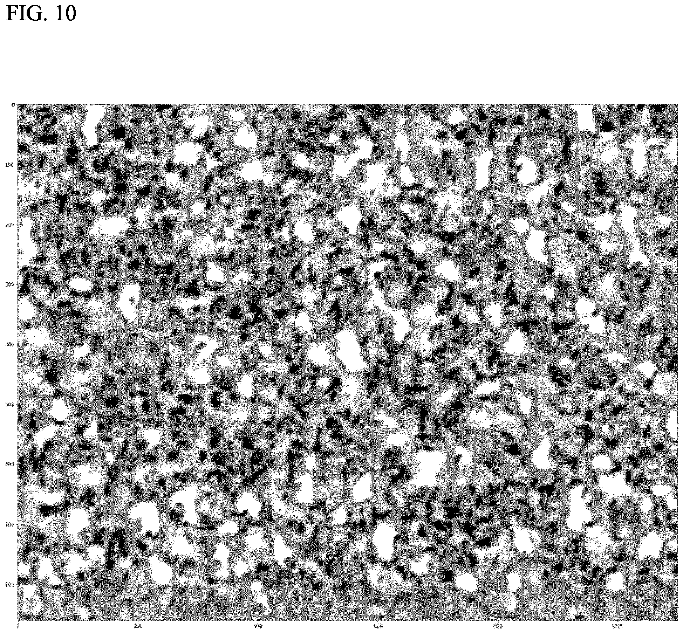

Fig. 6 is a diagram obtained by plotting data of combinations of the predicted values and the measured values, and is a diagram illustrating an example where a poor prediction image G3 and a good prediction image G4 are identified. As illustrated in Fig. 6, the identification unit 23 identifies an image with the largest error between the measured value and the predicted value as the poor prediction image G3 and identifies an image with the smallest error between the measured value and the predicted value as the good prediction image G4 from images almost identical in measured physical property value among the plurality of prediction target images. The "almost identical in measured physical property value" means that the measured physical property values are not exactly the same, but fall within a predetermined range as indicated by an area filled with gray in Fig. 6. The predetermined range can be appropriately set considering the dispersion level of the data. Fig. 7 is a diagram illustrating the poor prediction image G3 (SEM image). Fig. 8 is a diagram illustrating the good prediction image G4 (SEM image). Fig. 9 is a diagram illustrating, as an image, a feature map obtained from the poor prediction image G3 (SEM image). Fig. 10 is a diagram illustrating, as an image, a feature map obtained from the good prediction image G4 (SEM image). It is hard for anyone to understand, even looking at Figs. 7 to 10, a reason for a difference in prediction accuracy.

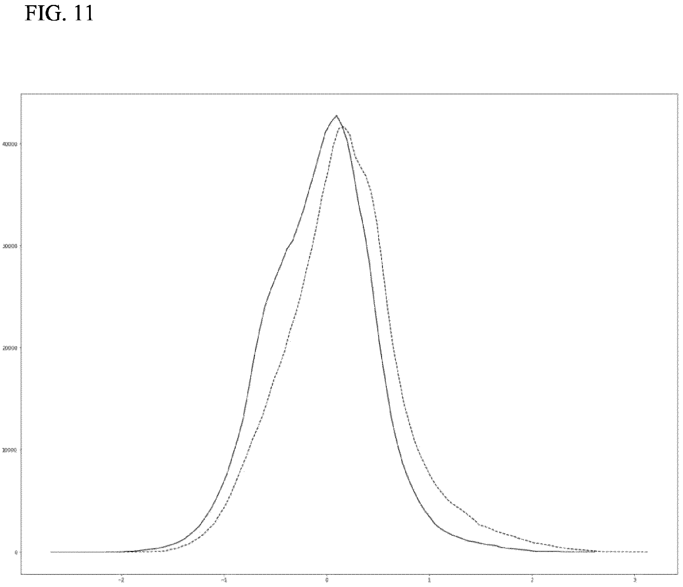

Fig. 11 is a diagram illustrating a frequency distribution (histogram) of a plurality of features constituting the feature map of the poor prediction image G3 and a frequency distribution (histogram) of a plurality of features constituting the feature map of the good prediction image G4. In Fig. 11, the horizontal axis represents a feature, and the vertical axis represents the number of pixels (that is, frequency). A solid line in Fig. 11 indicates the histogram of the poor prediction image G3, and a dashed line in Fig. 11 indicates the histogram of the good prediction image G4. Referring to Fig. 11, both the feature maps are similar in feature distribution, which is considered to indicate the feature of the SEM image of a copper surface, but are slightly different in feature distribution. In the present example, there is a large difference in frequency distribution in a range where the feature is greater than or equal to -1 and less than or equal to 1. Such a feature group (features greater than or equal to -1 and less than or equal to 1) were extracted as a feature group indicating a factor in poor prediction. Note that a feature group having a large difference as viewed with human eyes may be extracted, or may be automatically extracted by a program. Specifically, it is conceivable to calculate an integral value of a difference between two histograms for each closed region formed by the two histograms and extract a feature group in a range where the integral value is the largest.

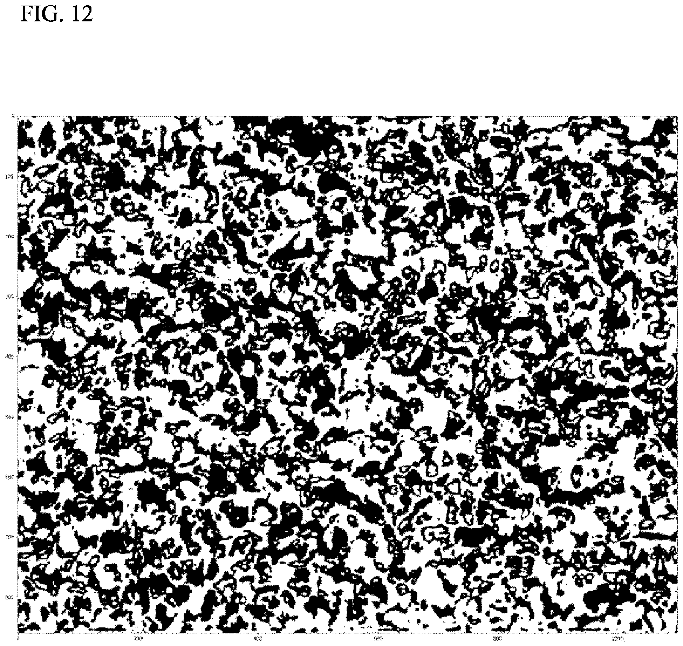

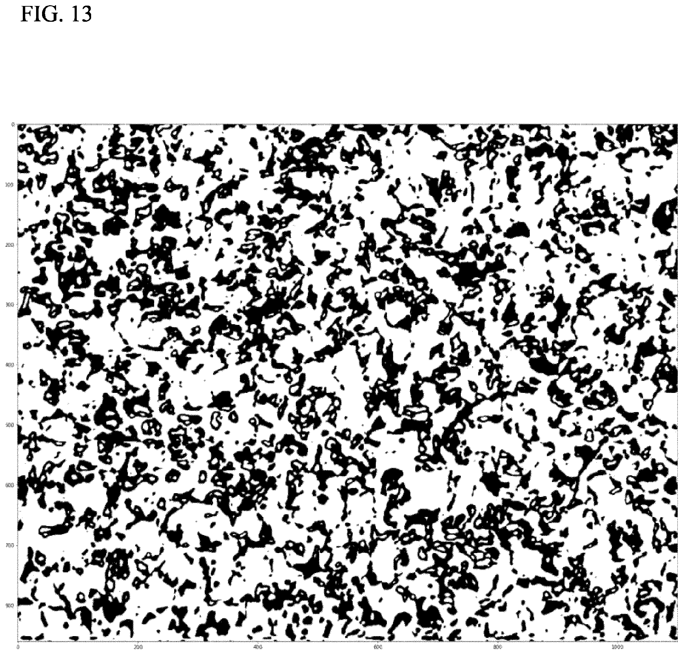

That is, the extraction unit 24 can extract a feature group representing the factor in poor prediction on the basis of the difference between the frequency distribution (solid line in Fig. 11) of the plurality of features constituting the feature map of the poor prediction image G3 and the frequency distribution (dashed line in Fig. 11) of the plurality of features constituting the feature map of the good prediction image G4. Fig. 12 is a diagram illustrating only the feature group extracted from the feature map of the poor prediction image G3. Fig. 13 is a diagram illustrating only the feature group extracted from the feature map of the good prediction image G4. In Figs. 12 and 13, the extracted feature portion is indicated in black, and the other portion is indicated in white. The feature maps are images identical in size or aspect ratio to the original poor prediction image G3 and the original good prediction image G4 in many cases, and each feature map can be used as it is or enlarged to maintain a positional relationship with the original image, and it can be understood that a portion of the original image in which the extracted feature group exists affects the prediction accuracy. Therefore, a result of comparing Figs. 12 and 13 with their respective original SEM images can be utilized in determining the factor in poor prediction, which may enable an explanation of the prediction accuracy.

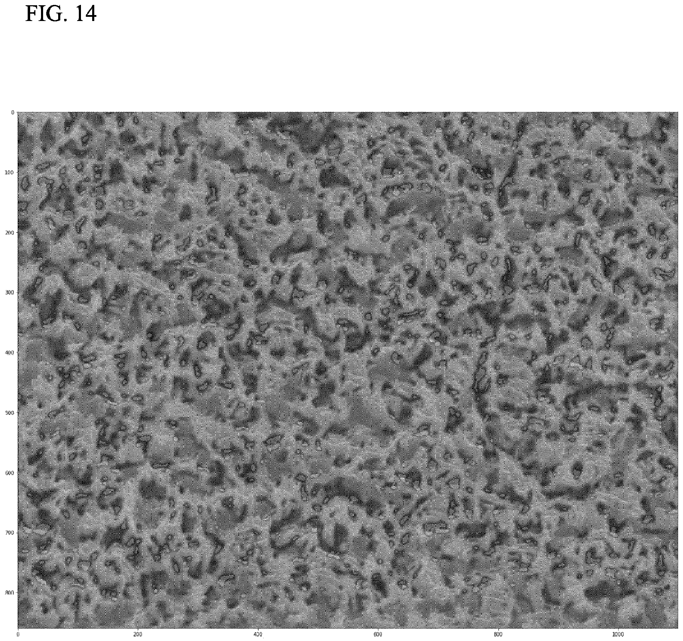

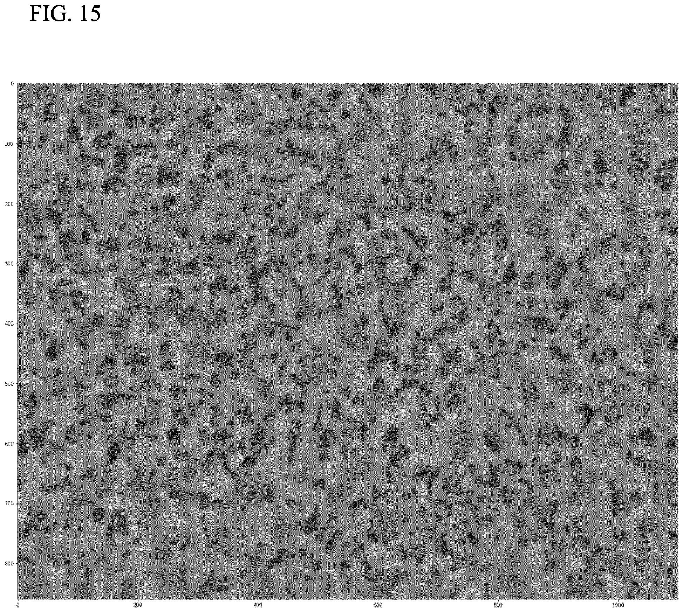

The superimposed image output unit 25 outputs a superimposed image that displays the position of the feature group extracted by the extraction unit 24 overlaid on at least one of the poor prediction image G3 or the good prediction image G4. Fig 14 is a diagram illustrating an example of a superimposed image showing the position of the extracted feature group overlaid on the poor prediction image G3. Fig 15 is a diagram illustrating an example of a superimposed image showing the position of the extracted feature group overlaid on the good prediction image G4. The position of the feature group may be indicated by any color or may be indicated by other marks.

This allows the position of the prediction target image and the position of the feature group to be visually recognized at a time, which is useful.

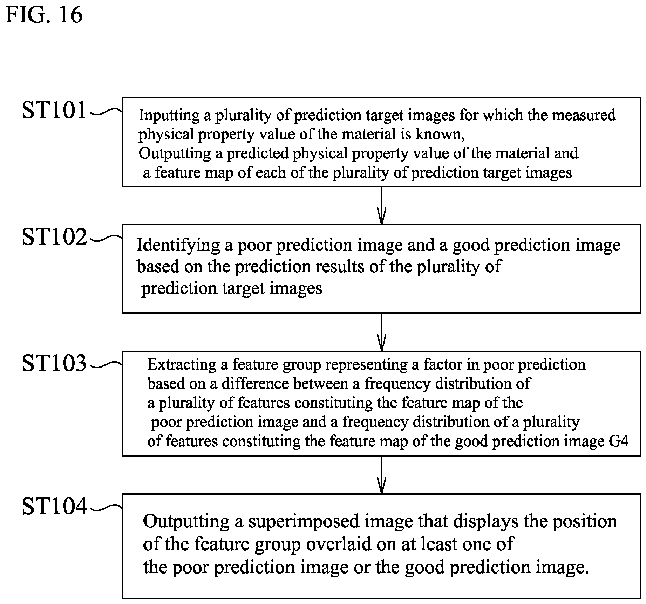

A method for predicting a physical property value of a material that is executed by the prediction system 2 of the second embodiment will be described with reference to Fig. 16.

First, in step ST101, the prediction unit 22 receives the input of a plurality of prediction target images for each of which the measured value of the physical property value of the material is known, and outputs a predicted value of the physical property value of the material and a feature map of each of the plurality of prediction target images.

In the next step ST102, the identification unit 23 identifies, on the basis of prediction results from images almost identical in measured physical property value among the plurality of prediction target images, the poor prediction image G3 with an error between the measured value and the predicted value greater than or equal to the first threshold and the good prediction image G4 with an error between the measured value and the predicted value less than or equal to the second threshold, the second threshold being smaller than the first threshold value.

In the next step ST103, the extraction unit 24 extracts a feature group representing the factor in poor prediction on the basis of a difference between the frequency distribution of the plurality of features constituting the feature map of the poor prediction image G3 and the frequency distribution of the plurality of features constituting the feature map of the good prediction image G4.

In the next step ST104, the superimposed image output unit 25 outputs a superimposed image that displays the position of the feature group overlaid on at least one of the poor prediction image G3 or the good prediction image G4.

As described above, the physical property value prediction method of the second embodiment may be executed by one or more processors, and may include: inputting, into a prediction model 21 including a feature map output unit 21a configured to output a feature map on a basis of an image obtained by imaging a material and a conversion unit 21b configured to convert the feature map into a physical property value of the material, and machine-learned to receive input of the image obtained by imaging the material as an explanatory variable and output the physical property value, a plurality of prediction target images for each of which a measured value of the physical property value is known and outputting a predicted value and a feature map of each of the plurality of prediction target images; identifying, on a basis of prediction results from images almost identical in measured physical property value among the plurality of prediction target images, a poor prediction image G3 with an error between the measured value and the predicted value greater than or equal to a first threshold and a good prediction image G4 with an error between the measured value and the predicted value less than or equal to a second threshold, the second threshold being smaller than the first threshold; and extracting a feature group representing a factor in poor prediction on a basis of a difference between a frequency distribution of a plurality of features constituting the feature map of the poor prediction image G3 and a frequency distribution of a plurality of features constituting the feature map of the good prediction image G4.

Accordingly, a feature group having a large difference between the frequency distribution of the plurality of features constituting the feature map of the poor prediction image G3 and the frequency distribution of the plurality of features constituting the feature map of the good prediction image is highly likely to represent the factor in poor prediction, so that the feature group can be utilized in determining the factor in poor prediction, and the possibility of enabling an explanation of the prediction accuracy can be provided.

Although not particularly limited, as in the second embodiment, the method may further including: outputting a superimposed image that displays the position of the feature group overlaid on at least one of the poor prediction image G3 or the good prediction image G4.

This enables the feature group to be visually recognized while superimposed on the poor prediction image or the good prediction image, which is useful.

Although not particularly limited, as in the second embodiment, the material to be imaged may be any one of a surface of a metal subjected to surface treatment, a surface coated with a coating material, a surface of a plated metal, a surface of a film, a paper surface, or a surface of a molded material. The above-described examples are preferred examples.

The system according to the second embodiment includes one or more processors that execute the above-described method.

The program according to the second embodiment is a program that causes one or more processors to execute the above-described method.

It is also possible to achieve the effects of the above-described method by executing such a program.

### [Modification]

(2-1) In the second embodiment, the superimposed image output unit 25 is provided, but the superimposed image output unit 25 can be omitted.

(2-2) The second embodiment has been described using the SEM image obtained by imaging a copper surface subjected to surface treatment and an example where peel strength is predicted, but the material to be imaged can be changed to any material as in the first embodiment. Further, the physical property value of the prediction target can be any value as in the first embodiment.

The structure employed in each of the above-described embodiments can be employed in any other embodiment. The specific configuration of each unit is not limited to the above-described embodiments, and various modifications can be made without departing from the gist of the present disclosure.

For example, the execution order of each processing such as operations, procedures, steps, and stages in the devices, systems, programs, and methods illustrated in the claims, the description, and the drawings may be any order unless the output of the previous processing is used in the subsequent processing. Even if the flows in the claims, the description, and the drawings are described using "first", "next", and the like for the sake of convenience, it does not mean that it is essential to execute in this order.

Each unit illustrated in Fig. 1 is implemented by a predetermined program executed by one or a plurality of processors, but each unit may include a dedicated memory or a dedicated circuit. In the system 1 (2) of the above-described embodiments, each unit is implemented by the single computer processor 1a (2a), but each unit may be distributed over and implemented by a plurality of computers or a cloud. That is, the above-described methods may be each executed by one or a plurality of processors.

The system 1 (2) includes the processor 1a (2a). For example, the processor 1a (2a) may be a central processing unit (CPU), a microprocessor, or other processing unit capable of executing computer-executable instructions. Further, the system 1 (2) includes the memory 1b (2b) for storing data of the system 1 (2). As an example, the memory 1b (2b) includes a computer storage medium, and includes a RAM, a ROM, an EEPROM, a flash memory or other memory technology, a CD-ROM, a DVD or other optical disc storage, a magnetic cassette, a magnetic tape, a magnetic disk storage or other magnetic storage device, or any other medium that can be used to store desired data and that can be accessed by the system 1.

### DESCRIPTION OF REFERENCE SIGNS

- G1: Prediction target image
- G2: Feature map
- 2: Prediction system
- 20: Image acquisition unit
- 21: Prediction model
- 21a: Feature map output unit
- 21b: Conversion unit
- 22: Prediction unit
- 23: Identification unit
- 24: Extraction unit
- 25: Superimposed image output unit

## Claims

1. A physical property value prediction method comprising:
inputting, into a prediction model including a feature map output unit configured to output a feature map on a basis of an image obtained by imaging a material and a conversion unit configured to convert the feature map into a physical property value of the material, and machine-learned to receive input of the image obtained by imaging the material as an explanatory variable and output the physical property value, a plurality of prediction target images for each of which a measured value of the physical property value is known and outputting a predicted value and a feature map of each of the plurality of prediction target images;
identifying, on a basis of prediction results from images almost identical in measured physical property value among the plurality of prediction target images, a poor prediction image with an error between the measured value and the predicted value greater than or equal to a first threshold and a good prediction image with an error between the measured value and the predicted value less than or equal to a second threshold, the second threshold being smaller than the first threshold; and
extracting a feature group representing a factor in poor prediction on a basis of a difference between a frequency distribution of a plurality of features constituting the feature map of the poor prediction image and a frequency distribution of a plurality of features constituting the feature map of the good prediction image.

2. The physical property value prediction method according to claim 1, further comprising outputting a superimposed image that displays the position of the feature group overlaid on at least one of the poor prediction image or the good prediction image.

3. The physical property value prediction method according to claim 1 or 2, wherein the material to be imaged is any one of a surface of a metal subjected to surface treatment, a surface coated with a coating material, a surface of a plated metal, a surface of a film, a paper surface, or a surface of a molded material.

4. A physical property value prediction system comprising:
a prediction unit configured to input, into a prediction model including a feature map output unit configured to output a feature map on a basis of an image obtained by imaging a material and a conversion unit configured to convert the feature map into a physical property value of the material, and machine-learned to receive input of the image obtained by imaging the material as an explanatory variable and output the physical property value, a plurality of prediction target images for each of which a measured value of the physical property value is known and output a predicted value and a feature map of each of the plurality of prediction target images;
an identification unit configured to identify, on a basis of prediction results from images almost identical in measured physical property value among the plurality of prediction target images, a poor prediction image with an error between the measured value and the predicted value greater than or equal to a first threshold and a good prediction image with an error between the measured value and the predicted value less than or equal to a second threshold, the second threshold being smaller than the first threshold; and
an extraction unit configured to extract a feature group representing a factor in poor prediction on a basis of a difference between a frequency distribution of a plurality of features constituting the feature map of the poor prediction image and a frequency distribution of a plurality of features constituting the feature map of the good prediction image.

5. The physical property value prediction system according to claim 4, further comprising a superimposed image output unit configured to output a superimposed image that displays the position of the feature group overlaid on at least one of the poor prediction image or the good prediction image.

6. The physical property value prediction system according to claim 4 or 5, wherein the material to be imaged is any one of a surface of a metal subjected to surface treatment, a surface coated with a coating material, a surface of a plated metal, a surface of a film, a paper surface, or a surface of a molded material.
